# EUROPEAN PATENT APPLICATION

(11) **EP 4 079 155 A1**
(43) Date of publication of application: **26.10.2022**
(21) Application number: 21170046.3
(22) Date of filing: 23.04.2021
(51) Int. Cl.: A01N 1/02, A61F 2/02, F25D 15/00, B01L 7/00

(54) **SYSTEM FOR THE HANDLING OF CELL TRANSPLANTS, AND COOLER, TRANSPLANT BASE AND POSITIONER APPLICABLE THEREIN, AND METHOD FOR THE COOLING OF CELL TRANSPLANTS**

(71) Applicant: Epiheart Oy, 00290 Helsinki (FI)
(72) Inventor: Kuuva, Aleksi, 00100 Helsinki (FI); Kronström, Kai, 02200 Espoo (FI); Kankuri, Esko, 00670 Helsinki (FI)
(74) Representative: Genip Oy

(57) **Abstract**

A system (1) for the handling of cell transplants is disclosed that comprises: (i) a continuously-operating cooler (10), which comprises at least one plate-like base (11), which comprises a cooled area (12); (ii) a sterile positioner (30); and (iii) a sterile cell transplant base (40). The sterile positioner (30) is arranged to align the sterile cell transplant base (40) relative to the continuously-operating cooler (10) so that the cell transplant base (40) comes at the cooled area (12). In a method for the cooling of cell transplants used in transplantation therapy, the system (1) is used so that the plate-like base is covered by a sterile cloth (20), after which the sterile positioner is placed over the plate-like base, after which the cell transplant base is placed in the positioner, whereby the cell transplant base is located at the cooled area and is coolable by the cooler.

## Description

### Field of invention

The invention is related to the field of medical technology, and specifically to the handling and cooling of cell transplants in a sterile environment.

### Technical background

In epicardial micrograft transplantation therapy, atrial appendage derived cells (AADC) are handled and transplanted in a therapeutical purpose back the cardiac area. The transplantation is typically performed using the patient's own cells (autologous graft), and the removal of the tissue and the fastening of the transplant take place within a single surgical operation. Tissue is taken from the atrial appendage for the procedure during the operation. This tissue potentially contains an abundance of cardiac stem cells (CSC) and other cells that support regeneration.

A tissue sample, which contains cells originating from the cardiac tissue (also AADC), is isolated into micrografts by, for example, mechanical rasping, which increases the surface area of the pieces of tissue. After this, a cellular micrograft is prepared from extracellular matrix (ECM), AADC and fibrin sealant, which bonds the micrograft to a thin extracellular matrix in a slow gelling process. The micrograft is sutured to the heart epicardially over an ischemic damage after performing anastomosis but before reperfusion, which means the restoring of blood circulation into the heart. (Nummi *et al.* 2017, Lampinen *et al.* 2017). Instead of fibrin sealant, other gelling material or applicable material that coagulates to some extent, such as autologous blood products, may also be used.

After preclinical animal studies, Nummi *et al.* (2017) planned and executed the first-in-man study. The recruitment of patients was started in February 2016, and a total of six patients were given an epicardial autologous atrial micrograft (AAM) in conjunction with coronary artery bypass graft (CABG). The therapy was considered to be safe and functional, and an indication of the thickening of the ventricular wall was also detected (waiting for publication, peer review in progress at the time of writing this, https://www.medrxiv.org/content/10.1101/2021.03.05.21252995v1) .

In a prior art arrangement, the re-sterilisable instruments are sterilised and packed in a transplant operation before the operation, and the other necessary equipment is gathered. In a prior art arrangement, a great number (as many as 23) of sterile instrument pockets would have to be opened at the beginning of the operation, and the instruments would also have to be laid on a table. This requires effort and above all time. This is just barely doable in a small scale, but when the procedure becomes more common, it cannot be assumed that so much time is spent on preparations. It is also possible that a coronary artery bypass operation has to be performed urgently, which reduces the time available for preparations even more.

In view of the success of epicardial autologous atrial micrograft therapy, it is important that the dying of the cells in the cell transplant can be restricted. This is usually sought by reducing the temperature of the cell transplant; however, without freezing the cell transplant. In therapies to date, a cold pack has been used for cooling the cell transplant.

### Objective of invention

The use of a cold pack is problematic. One problem is the thermal capacity of a cold pack. An operation may turn out to be more complicated, whereby its duration may be up to several hours. However, a small cold pack melts in such a period of time, as a result of which its cooling capability decreases. In order to slow down the melting of the cold pack, it must be implemented in such a way that the volume of heat required by melting is relatively large. Such an effective cold pack, however, easily freezes the cell transplant. On the other hand, reducing the volume of heat required by the melting of the cold pack deteriorates the cooling capability if the duration of the operation is extended. On the other hand, an increase in temperature also easily spoils the cell transplant.

Of course, there are continuously-operating coolers. Cryoplate 4X (https://www.gordiamkey.com/laboratory-coldplate/) offered by Alevax AB under the auxiliary business name GordiamKey comprises a cooling plate and is equipped with fans. Iceberg+ (https://www.cellpath.com/iceberg-cold-plate-230v-jri-0400-00a.html) of CellPath Limited comprises a cooling plate and is equipped with a compressor.

The cooler equipped with fans, referred to in the preceding text paragraph, is not applicable to the purpose, because the cooler would circulate air from the operating theatre onto the cell transplant that is on a sterile cloth and onto sterile instruments that are on a sterile base, which would cause a risk of contamination. The operation takes place in a sterile room, and the cell transplants need to be kept on a sterile base, which is on a sterile cloth.

Moreover, the cooler equipped with a compressor, referred to in the text paragraph preceding the preceding paragraph, would not be applicable to the purpose, because the cooler would have to be covered by a sterile cloth, which would lead to heat buildup and would burden the compressor. On the other hand, said cooler cannot be sterilised in a simple manner due to its structure (ventilation grill etc.), which is why it is not be applicable to use in a sterile environment (such as in an operating theatre environment on a sterile cloth).

The primary objective of the invention is to improve the chances of success of transplantation therapy and especially of epicardial micrograft transplantation therapy.

This objective can be solved by means of a system according to the independent claim for the handling of cell transplants using the system, by means of a cooler and cell transplant base applicable to the cooling of cell transplants according to the parallel independent claims, by means of a positioner applicable to the handling system of cell transplants and by means of a method for the cooling of cell transplants used in transplantation therapy, most advantageously in epicardial micrograft transplantation therapy.

### Advantages of invention

The system for the handling of cell transplants comprises:
(i) a continuously-operating cooler, which comprises at least one plate-like base, which comprises a cooled area;
(ii) a sterile positioner; and
(iii) a sterile cell transplant base.
Moreover, the sterile positioner is arranged to align the sterile cell transplant base relative to the continuously-operating cooler so that the cell transplant base comes at the cooled area.

In this way, it is possible to align the cell transplant base at the cooled area. In this way, it is possible to improve the likelihood of success of micrograft transplantation therapy.

The advantage of the invention is noticeable especially when the system is used with a sterile cloth or when the system also comprises a sterile cloth: In the operating theatre, the plate-like area is covered by a sterile cloth. The sterile positioner is arranged to align the cell transplant base at the cooled area (while the plate-like area is covered by a sterile cloth) so that the sterile cell transplant base comes against the sterile cloth and at the cooled area.

The sterile cloth to be placed on the plate-like base obstructs view of the exact location of the cooled area. In this case, even though the cooled base would be marked with a visible identifier, the sterile cloth would cover such an identifier, too. The visual obstruction caused by the sterile cloth complicates the positioning of the cell transplant base onto the cooled area.

In the system, alignment is implemented advantageously by means of the coding (which is advantageously a rotation coding, in other words the parts match each other in a specific position or positions only), shape, at least one opening and/or dimensioning of the positioner and the plate-like base and/or cell transplant base, adapted to each other. Such coding based on shape prevents the cell transplant base from ending up at such a location on the planar base that is not a coolable area.

The cooler most advantageously comprises a cooling and adjustment system, which is configured to keep said at least one plate-like base within a temperature range that is suitable for the cell transplants in order to control the dying of cells. Alternatively to this or in addition to this, the cooling and adjustment system may be configured to keep said at least one plate-like base within a temperature range that is suitable for the cell transplants in order to control the gelling speed of the extracellular matrix of the transplants. By means of a cooler implemented in this way, it is possible to improve the likelihood of success of micrograft transplantation therapy.

The positioner is most advantageously placeable over the plate-like part. In this case, the positioner has advantageously a number of alignment edges, which are most preferably edged or contain a protrusion, and these have a joint effect with the alignment edges of the plate-like part corresponding to these in order to secure the position of the positioner. This is an effective way of preventing the shifting of the positioner so that the cell transplant base would end up at such a location on the planar base that is not a coolable area.

The positioner advantageously comprises a positioning hole, which, when the positioner is installed over the plate-like part, is aligned over the cooled area and at which the cell transplant base is placeable for cooling. This is an effective way of preventing the shifting of the cell transplant base to such a location on the planar base that is not a coolable area. In this way, it is also possible to accelerate the transfer of heat from the cooled cell transplant base to the cooled area, when the positioner is not in between.

The positioner and the cell transplant base are most advantageously separate parts of the system. This enables the use of the positioner in an operating theatre also if the cell transplant base has to be moved to another place. On the other hand, according to an alternative aspect, the positioner is integrated into the cell transplant base. This prevents the positioner and the cell transplant base from coming apart from each other and potentially getting lost.

According to one aspect of the invention, the cooler applicable to the cooling of cell transplants is suitable for use in a system of the invention presented above. In this case, the cooler comprises at least one plate-like base, which comprises a cooled area. Moreover, the cooler comprises a cooling and adjustment system for the cooling of said cooled area, which cooling and adjustment system is arranged to transfer heat from the cooled area to the lower side of the plate-like base. This may be done with blowers, for example. The transfer of heat (by blowing, for example) to the lower side of the plate-like base is a special arrangement, because heat tends to be conducted or transferred (and when blown be transferred in air) upwards, in this way actually heating the plate-like base and the cooled area to some extent.

By doing this, the actual advantage to be achieved is, however, that when heat is transferred from the cooled area to the lower side of the plate-like base, heat is also essentially transferred to the lower side of the sterile cloth (which covers the plate-like base). This facilitates the avoidance of the contamination of the cell transplant base and of the transplants possibly located on it, especially if heat is ultimately transferred to the air by a number of blowers. In this case, the blowers may most advantageously be made to blow air from the cooler specifically to the lower side of the plate-like base. In this case, the blowing direction of the blowers can be selected so that the blowers do not cause a contamination risk or cause at the most an increase in contamination risk at the most to an acceptable level.

The cooling and adjustment system is most advantageously configured to control the cooling of the cooled area and to adjust its temperature, advantageously by keeping said at least one cooled area within a temperature range that is suitable in terms of the cell transplants in order to control the dying of cells and/or to control the gelling speed of the extracellular matrix of the transplants. In this way, it is possible to improve the likelihood of success of micrograft transplantation therapy.

The cooling and adjustment system most advantageously comprises a number of thermoelectric elements or Peltier elements, the cold side of which is arranged to refrigerate said at least one cooled area and where the heat produced by the warm side of the elements is arranged to be transferred via a liquid cooling circuit to the air or other fluid. A significant advantage in terms of a medical technology device is that the thermoelectric element (or Peltier element) does not have any moving parts. In this case, it is easier to assemble the cooling and adjustment system so that it can be sterilised if necessary. This also enables the implementation of the cooler as sterile or sterilisable.

The cooler advantageously comprises a number of blowers, which are arranged to remove heat from the liquid cooling circuit and are placed beneath the plate-like base. The blowers are simple yet efficient components. In practice, in this case the cooler also has a liquid-air heat exchanger. According to one aspect, the blowers and the liquid-air heat exchanger are arranged at least as one removable module. This enables that a contaminated cooler can be made clean and even sterile, when the removable module(s) is/are removed, and the remaining part of the cooler is cleaned or sterilised. After this, clean or sterilised removable module(s) is/are installed in the device. In this way, it is possible to reuse (if necessary in a clean or sterile manner) an expensive cooler that contains more complex components, and the blower and the liquid-air heat exchanger modules(s), which are more advantageously replaceable but more difficult to clean, can be replaced with new one(s) .

The cooling and adjustment system advantageously comprises at least two temperature sensors, which are arranged to monitor the temperature of the cooled area (or of a thermal conductor - such as a thermal conduction plate - that is in a fixed thermal contact with the cooled area. The monitoring of the temperature of the cooled area can hence be implemented either directly or indirectly. Indirect temperature monitoring is very advantageous especially if the cooling and adjustment system is implemented by means of a number of thermoelectric elements, because in this case it is important that there is good contact between the thermoelectric element and the cooled area. For this purpose, the thermal conductor (such as a thermal conduction plate) in the cooled area may comprise an extension, which extends in the lateral direction beyond the footprint of the thermoelectric element. The extension may also be folded downwards, and most advantageously the measurement is carried out inside the fold, in which case it is possible to reduce the measuring error caused by ambient temperature. In addition to this, the cooling system most preferably comprises an operating logic implemented on a main circuit board, for example (controlled by a microcontroller or processor, for example), which uses a measuring algorithm to interpret the readings provided by the sensors and reacts, when necessary, to a potential malfunction or deviation by means of warning sounds or lights. A malfunction or deviation here means a deviation from the target adjustment value of the measuring algorithm. The operating logic is advantageously calibratable. In addition to this or as an alternative to this, the operating logic may contain a correction algorithm for calculating the temperature specifically for the cooled area. The need for a correction algorithm is greater than if the temperature sensors are located father away.

The cell transplant base according to one aspect of the invention is intended for use in the above-described system according to the invention. The cell transplant base has a cup-like recess and a bottom that is essentially flat at least in some respect, for the transfer of heat from the cell transplant base via a sterile cloth to the cooled area. The cap of a dome, for example, is not essentially flat. In some respects, essential flatness requires in practice that the total area of the bottom of the cell transplant base and of a plane that intersects it is at least 1 cm², most advantageously at least 4 cm² (for example, 6 - 12 cm²) .

The cell transplant base is of metal or contains metal or some other material that conducts heat well, most advantageously so that the cell transplant base has a thermal conductivity of > 14 W / (m • K), and best so that the cell transplant base is of or contains steel - such as stainless steel - or aluminium. This enables the rapid cooling of the transplants.

The mass of the cell transplant base is most advantageously < 200 g, more advantageously 30 - 150 g, best 35 - 55 g. This enables the rapid cooling of the transplants.

The cell transplant base most advantageously comprises a cover part comprising a cup-like recess, and a body. The body and cover part may have a flange-like part, which is at least partly overlapping, for receiving heat from the cover part to the body. The flange-like part most advantageously encircles the cup-like recess. In addition to this or as an alternative to this, the cell transplant base may have a three-dimensional shape or protrusion that facilitates the handling of the cell transplant.

The positioner according to one aspect of the invention, applicable to the cell transplant handling system, is intended to be placed on a sterile cloth to be placed on the plate-like base in the above-described system. The positioner is arranged to position the cell transplant base at the cooled area so that the cell transplant base and the cooled area exert a force on the sterile cloth from the opposite sides of the sterile cloth. By means of the positioner, it is possible to press the sterile cloth flat and keep it flat, which facilitates the use of the cell transplant base, when it is possible to avoid a possible poorer thermal conductivity caused by the folding of the sterile cloth. This contributes to the cooling of the transplant base and to keeping it cool.
This is most advantageously accomplished by the shape: The positioner is so shaped that it allows both the gravitational force of the cell transplant base to be essentially directed to the cooled area and, correspondingly, the travel of the support force, which comes to the cell transplant base via the cooled area and which is opposite to the gravitational force, via the sterile cloth. In practice, the simplest way to implement the positioning is to provide the transplant base with a positioning hole, which is dimensioned to be slightly bigger than the diameter of the cell transplant base.

The positioner may also contain other functionalities such as cup holders, stands for one or more sample tubes, instrument stands or worktops. This may be advantageous, because in this case the work environment can be standardised, which contributes to the minimisation of human errors. It may also be advantageous from the point of view of manufacturing costs to include functionality into the positioner.

Moreover, the positioner may be arranged to be part of a kit, which includes sterile tools for the handling of cell transplants and/or providing epicardial autologous atrial micrograft therapy. This facilitates the commissioning of the system, for example when epicardial autologous atrial micrograft therapy is given in connection with an urgent coronary artery bypass operation, in which case there is little preparation time available. For the same reason, the positioner may be arranged to be part of a functional worktop and/or the positioner may include at least one cup, tube stand or cup holder.

It is sufficient for the commissioning of the system when preparations are being made for an operation that a sterile cloth is placed on the (possibly cleaned and/or sterilised) plate-like area of the cooler, and the positioner is placed on the sterile cloth.

The positioner is arranged to align the sterile cell transplant base relative to the continuously-operating cooler so that the cell transplant base comes at the cooled area. The cooler is switched on. If the kit includes the necessary sterile tools for the handling of cell transplants and/or providing epicardial autologous atrial micrograft therapy, optionally including the sterile cell transplant base (which, of course, may also be in its own sterile packaging), it is possible to put into order several issues that are critical to the success of epicardial autologous atrial micrograft therapy.

In the method according to one aspect of the invention for the cooling of cell transplants used in transplantation therapy, most advantageously in epicardial micrograft transplantation therapy, the system as described above is used so that the plate-like base is covered by a sterile cloth, after which the sterile positioner is placed over the plate-like base, after which the cell transplant base is placed in the positioner, whereby the cell transplant base is located at the cooled area and is coolable by the cooler.

### List of drawings

The system, positioner, cell transplant base and cooler are presented below in more detail in the application example presented in FIG 1 - 8. Of the drawings:
- FIG 1: shows an exploded view of the parts of the system;
- FIG 2: shows an exploded view of the cooler;
- FIG 3: shows the cell transplant base (perspective view);
- FIG 4: shows the cell transplant base (viewed from above);
- FIG 5: shows the cell transplant base (viewed from a side);
- FIG 6: shows an exploded view of the cell transplant base;
- FIG 7: shows the positioner (perspective view); and
- FIG 8: shows time series of temperatures measured when using the system.

The same reference numbers refer to the same technical parts in all FIGs.

### Detailed description of the invention

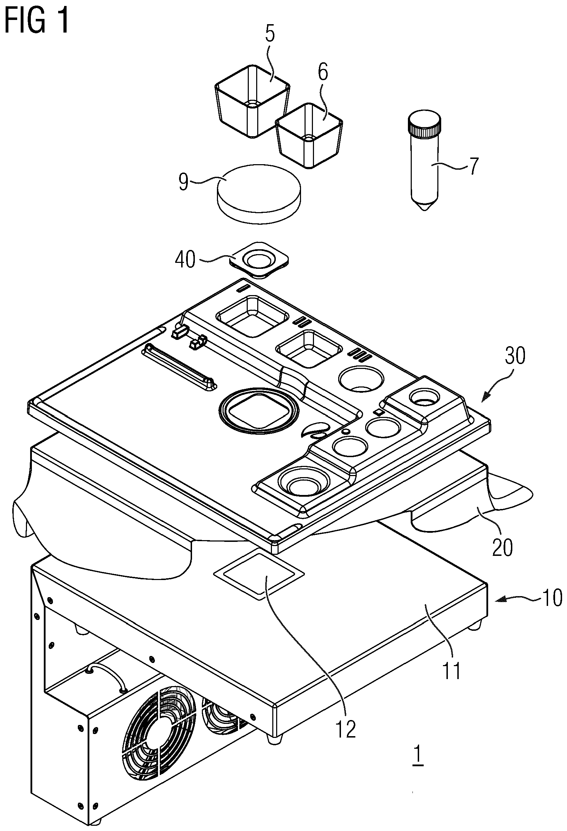

FIG 1 is an exploded view of system 1 for the handling of cell transplants. The system 1 comprises a continuously-operating cooler 10. The cooler 10 comprises at least one plate-like base 11, which comprises a cooled area 12. Moreover, the system 1 comprises a sterile positioner 30 and a sterile cell transplant base 40.

The sterile positioner 30 is arranged to align the sterile cell transplant base 40 relative to the continuously-operating cooler 10 so that the cell transplant base 40 comes at the cooled area 12.

In use, the system 1 is used with a sterile cloth 20. This can be packed, for example, into a kit with the positioner 30. In addition to this or as an alternative to this, the kit includes not only the positioner 30 but also sterile tools for the handling of cell transplants and/or providing epicardial autologous atrial micrograft therapy. These tools can include, for example, a number of separate cups 5, 6, a number of centrifugal tubes 7, cover 9.

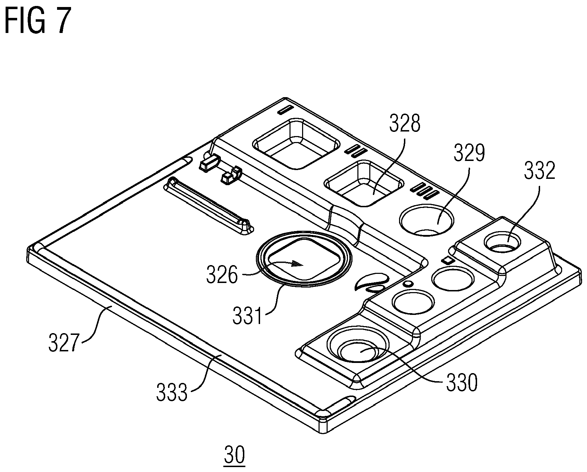

FIG 7 shows a perspective view of the positioner 30. The positioner 30 is adapted to position the cell transplant base 40 at the cooled area 12 so that the cell transplant base 40 and the cooled area 12 exert a force on the sterile cloth 20 from the opposite sides of the sterile cloth 20.

The positioner 30 is so shaped that it allows both the gravitational force of the cell transplant base 40 to be essentially directed to the cooled area 12 and, correspondingly, the travel of the support force, which comes to the cell transplant base 40 via the cooled area 12 and which is opposite to the gravitational force, via the sterile cloth 30. For this, the positioner 30 most advantageously has a transplant base positioning hole 326.

The positioner 30 can also have a cover positioner 331. This is advantageously a groove or rebate, which can be implemented around the transplant base positioning hole 326, possibly so that the grip edge of the cell transplant base fits inside the circumference defined by the positioner 331. The cover 9 can be used for covering the cell transplant base 40 in order to reduce the risk of contamination, to reduce temperature gradients and to prevent the drying of the cell transplants.

The positioner 30 can also have a number of cup holders 328, a number of cups 329 integrated into the positioner, a stand 332 for centrifugal tubes and a number of tissue processing areas 330. The tissue processing areas 330 are used, for example, for slicing the cell transplants or for working on the cell transplants.

In accordance with the principle indicated by FIG 1 (line marked with a broken line), the sterile positioner 30 is arranged to align the cell transplant base 40 at the cooled area 12 while the plate-like area 11 is covered by a sterile cloth 20 so that the cell transplant base 40 comes against the sterile cloth 20 and at the cooled area 12.

The alignment is carried out by means of the coding, shape, openings (one or more openings) and/or dimensions of the positioner 30 and the plate-like base 11 and/or the cell transplant base 40, adapted to each other.

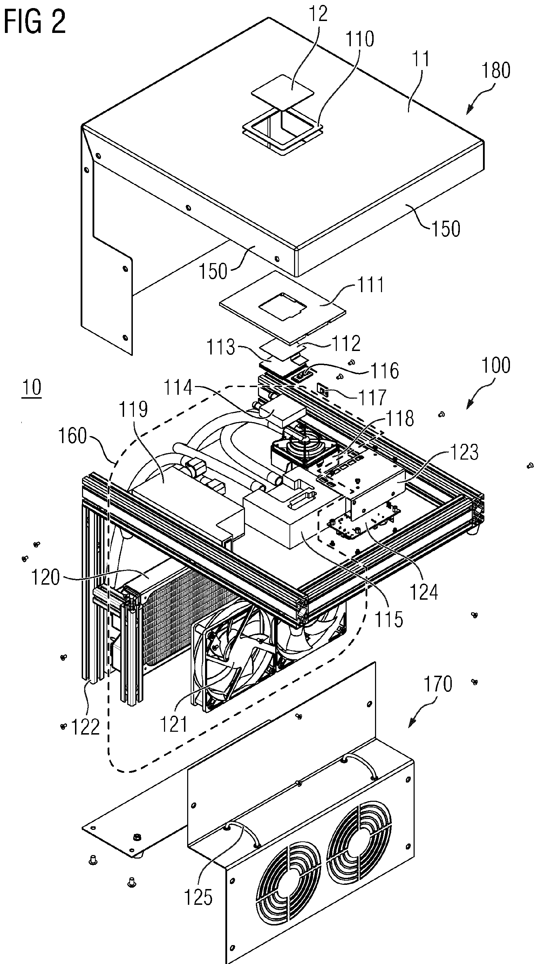

FIG 2 shows an exploded view of the cooler 10. The cooler 10 comprises, in addition to the above-presented at least one plate-like base 11 (which comprises the cooled area 12) also a cooling and adjustment system 100, which is most advantageously built into a device casing. The cooling and adjustment arrangement 100 is implemented to cool said cooled area 12, and is arranged to transfer heat from the cooled area 12 to the lower side of the plate-like base 11.

The device casing comprises a bottom part 170 and a top part 180. The plate-like base 11 and the cooled area 12 are part of the top part 180 of the device casing. Of course, the device casing can be segregated from the plate-like base 11, but segregation would only increase the need for cleaning, disinfection and possibly also sterilisation due to the extra break on the surface.

The cooling and adjustment system 100 is configured to keep said at least plate-like base 11 within a temperature range that is suitable in terms of the cell transplants in order to control the dying of cells and/or to keep said at least plate-like base 11 within a temperature range that is suitable in terms of the cell transplants in order control the gelling speed of the extracellular matrix of the transplants.

In order to intensify cooling, the cooled area 12 is insulated from the plate-like base 11 by means of, for example, insulation forming 110.

When the positioner 30 is placed over the plate-like base 11, a number of alignment edges 327 of the positioner 30 (which alignment edges 327 are edged and contain a protrusion in the cases of FIG 2 and 7) have a joint effect with the corresponding alignment edges 150 of the plate-like base 11 in order to secure the position of the positioner 30. The alignment edges 327 most advantageously encircle the entire plate-like base 11, in other words the alignment edges 327 are arranged in the lateral direction on each edge of the plate-like base 11.

The positioner 30 comprises a positioning hole 326, which, when the positioner 30 is installed over the plate-like part 11, is aligned over the cooled area 12 and at which the cell transplant base 40 is placeable for cooling.

The cooling and adjustment system 100 comprises a number of thermoelectric elements (or Peltier elements) 113, the cold side of which is arranged to refrigerate said at least one cooled area and where the heat produced by the warm side of the elements is arranged to be transferred via a liquid cooling circuit 160 to the air or other fluid.

In the case of FIG 2, the cold side of the thermoelectric element 113 is connected to the cooled area 12 by means of a thermal conduction plate 112. The thermal conduction plate 112 used is, for example, a copper plate. It is important for the conduction of heat that the surfaces are in a close contact with each other. This can be ensured by means of a support insert 111. The support insert 111, the thermal conduction plate 112 and the thermoelectric element as well as the cold side temperature sensors 116 (which are at least duplicated so that the failure of one temperature sensor can be detected) and the warm side temperature sensor 117 (which can be at least duplicated) are glued to each other and fastened from below tightly against the cooled area 12.

It is to be noted that the cold side temperature sensors 116 cannot be installed over the thermoelectric element 113. This is why the cold side temperature sensors 116 are installed on the thermal conduction plate 112 to be installed between the cooled area 12 and the thermoelectric element 113. For this, the edge of the thermal conduction plate 112 can be bent so that that an installation surface can be provided for the cold side temperature sensors 116.

Thermal adhesive is most advantageously used for gluing so that sufficient thermal conduction can be ensured.

The measuring method causes a delay and also a systematic error in the determination of the temperature of the cooled area 12. However, the error is of a constant magnitude. This is why it is possible to correct the error and delay by means of program and to calculate a corrected temperature of the cooled area 12. For the determination of an error with a constant magnitude, the temperature of the cooled area 12 is measured with an external thermometer, and the calculation unit located on the main circuit board 124 is programmed to take into account the calibration thus obtained.

In tests performed, the removal of heat from the warm side of the thermoelectric element 113 turned out to be insufficient if the warm side is not equipped with separate cooling. There was heat buildup inside the device casing. If the cooling of the thermoelectric element 113 is implemented by means of air cooling, in other words by increasing air flow beneath the thermoelectric element 113, two problems emerge: The first problem is that the sound level increases due to the resonance caused by air with the plate-like part 11 (buzzing sound that can be heard), which impairs the coping and concentration of the personnel participating in an operation.

Another and even more significant problem is that too great an air flow comes around the plate-like part 11, which causes a risk of contamination. The air flow causes the fluttering of the edge of the sterile cloth 20, which sets in motion secretory products that come on clothing during an operation, and the secretory products may be carried as aerosols or droplets also towards the transplant base 40.

For this reason, it was decided to carry out the cooling of the thermoelectric element 113 by means of a liquid cooling circuit 160. The liquid cooling circuit 160 is cooled by means of a number of blowers 121, which are arranged to remove heat from the liquid cooling circuit 160 and placed beneath the plate-like base 11. By means of the liquid cooling circuit 160, it is possible in practice to increase the distance of the blowers 121 from the cooled area 12 in the vertical direction in an almost arbitrary manner. The only things that are needed are a pump 118, a tank 119, a cooler 120 and a piping or hose system, and of course a heat carrier liquid (such as water, for example distilled water, but more preferably biocompatible antifreeze fluid for corrosion prevention) in the tank 119 and piping. After this, the system is vented. To facilitate the filling of the system, the liquid circulation has a ball valve with T-branches on both sides.

The heat exchanger 114 of the liquid cooling circuit is against the warm side of the thermoelectric element 113, most advantageously so that both are located in a recess that is made in the insulation piece 115 and that is open from the upper side. The hose system is connected to the pump 118, which pumps cooling liquid to the cooler 120 of the liquid cooling circuit. A number of blowers 121 are arranged to cool the cooler 120.

The frame 122 is dimensioned suitably so that the blowing provided by the blowers 121 can reach far enough beneath the cooled area 12.

We discovered in tests performed by us that the commercially available tankless cooling systems that we experimented with did not provide sufficient cooling efficiency. A tankless cooling system would of course have been ideal in terms of the mass and space requirement of the system. Moreover, we discovered that with time, the piping / hose system of the cooler 10 was bleeding air, which disturbed the operation of the pump. The hose system in the prototype was of silicone tubing, which allows the wall of the hose to permeate air quite well. In order to reduce the need for maintenance, we replaced the piping / hose system of the cooler with one that permeates little air, and added a liquid tank 119.

The sterile cloth 20 impairs the air flow. Moreover, no air comes through the plate-like base 11. In order to improve the cooling efficiency, there are therefore at least two blowers 121. In this case, one blower is operated advantageously in a push-pull mode, in other words one blower is used for blowing air to the first direction (towards the cooler 120) and the other blower is used for blowing air to the second direction, in other words away from the cooler, when the blowers are located side by side. Of course, the blowers can be placed on opposite sides of the cooler 120, in which case both blowers would be programmed to blow towards the cooler 120.

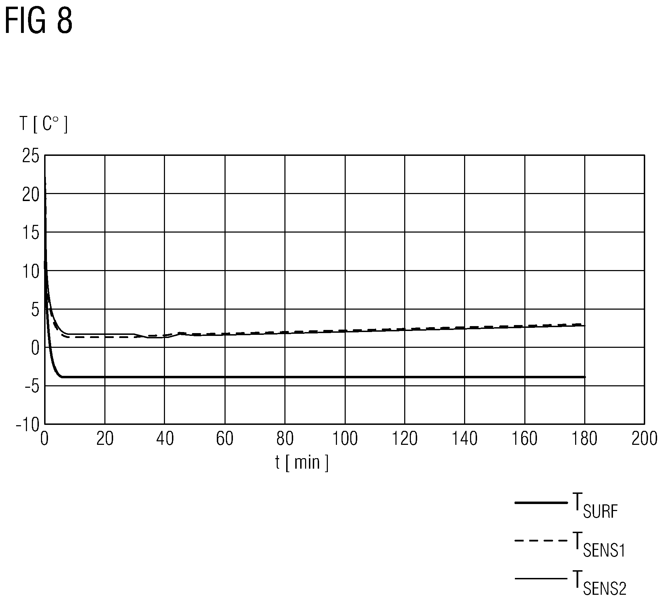

The system 1 and the functioning of the cooler 10 were tested. The results are presented in FIG 8. T_{SENS1} and T_{SENS2} are readings of the cold side temperature sensors 116. T_{SENS1} is the temperature of the cooled area 12, corrected in the above-presented manner. T_{SURF} reached the temperature of -4°C in five minutes and kept this temperature throughout the entire test measurement of 3 h.

The readings T_{SENS1} and T_{SENS2} of the temperature sensors 116 stabilised at 1.5°C, but they exhibited a trend that rose towards the end. This is due to a small heat buildup inside the device casing. It is possible to eliminate the rising trend (in other words, it is possible to better stabilise the temperature), if the thermoelectric element 113 is not operated at full efficiency and/or if the temperature sensors 116 are insulated thermally from the lower and lateral directions, for example by means of a piece of thermal insulation material (insulation piece 115).

In an actual operating situation, the thermoelectric element 113 is not operated at 100% efficiency all of the time, because the temperature of the cell transplant base 40 must not go below 0°C, not at least for an uncontrollably long period of time.

Because of the adjustment of the temperature of the cooled area 12, the thermoelectric element 113 is controlled by means of a pulse width modulated (PWM) signal. Our equipment had a PWM signal of 490 Hz, which we ran from a microcontroller on an Arduino^{®} (trade mark owned by Arduino SA in the EU and possibly elsewhere) main circuit board 124 to a transistor (placed on the main circuit board 124). Some other type of microcontroller or more generally a control component could have been used, and these could have been placed also otherwise. The transistor was added on the main circuit board 124 to feed a voltage of 12 V to the thermoelectric element 113. The PWM control does not decrease the reliability of the thermoelectric element 113, but it has been stated (Nagy and Roman 1999) to decrease the poor efficiency of the Peltier element, which is bad to begin with. In our prototype, the PWM signal was not equalised in any way, because it was noticed that even an unequalised signal was sufficient to control cooling.

The tests performed with the prototype in this way indicated that it is possible to use the cooler 10 to implement liquid cooling of the thermoelectric element 113 in the cooler with closed circulation. The cooling of the cooling liquid of the liquid cooling circuit can be implemented either with air cooling by using a liquid-liquid heat exchanger, for example from a water pipe to a sewer. Of these two, the air-cooled arrangement can be expected to stabilise the temperature better, because the goal is to keep the temperature of air in operating theatres at a constant temperature, but the temperature of water obtained from a water pipe may change based on the time of the day or the season of the year, for example due to the radiation impact of the sun (sun heats walls).

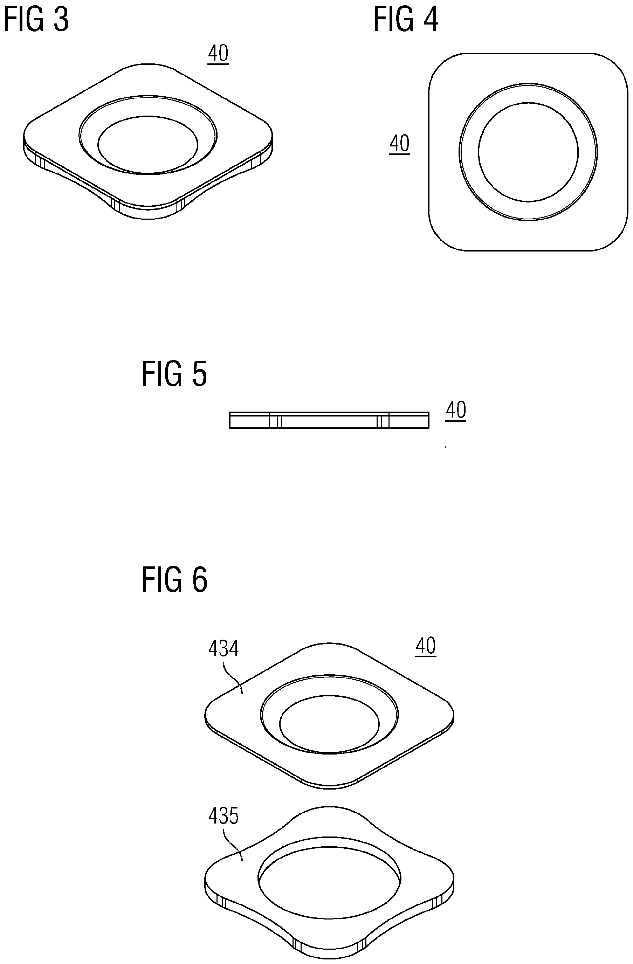

FIG 3 - 5 show a cell transplant base 40 suited to the cooling of cell transplants, which cell transplant base 40 is intended to be used in the system 1. The cell transplant base 40 has a cup-like recess and a bottom that is essentially flat at least in some respect, for the transfer of heat from the cell transplant base 40 via the sterile cloth 20 to the cooled area 12 as efficiently as possible. There are flanges on the sides of the cell transplant base 40 to enable a good grip.

The cell transplant base 40 used can be a re-sterilisable or disposable (but still sterilisable) item made of stainless steel, which has a cup-like recess for receiving an ECM transplant. The cooling of such a relatively large steel item consumes relatively much energy and takes a long time.

In order to reduce the mass to be cooled and to accelerate cooling, it is advantageous to implement the cell transplant base 40 in two or more parts (cf. FIG 6): the cover part 434 of the cell transplant base, in other words the cup-like top part can be implemented, for example, by punching a plate (for example steel plate, most preferably of stainless steel). Punching is carried out most advantageously so that the bottom of the cup of the cover part 434 becomes as flat as possible. After this, the cup-like cover part 434 is fastened, for example by gluing, to an essentially flat body 435, which has a hole. The diameter of the hole is most advantageously a little larger than that of the cup-like recess.

As a result of such a structure, the area of the bottom of the body 435 can be in contact (through the sterile cloth 20) to the cooled area 12. The body 435 cools the cover part 434 through the flange. In this way, it is possible to intensify the cooling of the cell transplant base 40, as compared to a situation where cooling was carried out merely by means of the contact of the bottom of the cup-like recess. In this way, the bottom of the cup-like recess can also be carried out in a way other than as flat. On the other hand, in this way it is possible to reduce the accuracy required from the punching, which enables a facilitated technical implementation of the manufacture of the cell transplant base 40.

In order to reduce the mass to be cooled and to accelerate cooling, it is also possible to use a disposable (but still sterilisable) or re-sterilisable cell transplant base, which is, for example, partly or completely made of aluminium. Aluminium is easily workable by milling, for example (possibly in a CNC machining system).

The cell transplant base 40 is of metal or contains metal or some other material that conducts heat well, most advantageously so that the cell transplant base 40 has a thermal conductivity of > 14 W / (m · K). Most advantageously, the cell transplant base 40 is of or contains steel (such as stainless steel) or aluminium. The biocompatibility of stainless steel speaks for the use of stainless steel at least in the surface part.

The mass of the cell transplant base 40 is most advantageously < 200 g, more advantageously 30 - 150 g, best 35 - 55 g.

In accordance with the method, in transplantation therapy, most advantageously in epicardial micrograft transplantation therapy, for the cooling of the cell transplants used, in which method: a system (1) according to any one of the claims 1 9 is used so that the plate-like base (11) is covered by a sterile cloth (20), after which the sterile positioner (30) is placed over the plate-like base (11), after which the cell transplant base (40) is placed in the positioner (30), whereby the cell transplant base (40) is located at the cooled area (12) and is coolable by the cooler (10).

The invention should not be understood to be limited only by the below claims, but the invention is to be understood to include all their legal equivalents and the combinations of the embodiments presented.

### List of reference numbers used

- 1: system for the handling of cell transplants
- 5: separate cup
- 6: separate cup
- 7: centrifugal tube
- 9: cover
- 10: continuously-operating cooler
- 11: plate-like base
- 12: cooled area
- 20: sterile cloth
- 30: sterile positioner
- 40: sterile cell transplant base
- 100: cooling and adjustment system
- 110: insulation forming
- 111: support insert
- 112: thermal conduction plate
- 113: thermoelectric element / Peltier element
- 114: heat exchanger of liquid cooling circuit
- 115: insulation piece
- 116: cold side temperature sensors
- 117: warm side temperature sensor
- 118: pump of liquid cooling circuit
- 119: tank of liquid cooling circuit
- 120: cooler of liquid cooling circuit
- 121: blowers of liquid cooling circuit
- 122: frame
- 123: fastening of main circuit board
- 124: main circuit board
- 125: storage fastening of power cable
- 150: alignment edge of plate-like base
- 160: liquid cooling circuit
- 170: lower part of device casing
- 180: upper part of device casing
- 326: positioning hole of transplant base
- 327: alignment edge of positioner
- 328: cup holder
- 329: cup integrated into positioner
- 330: tissue processing area
- 331: cover positioner
- 332: stand for centrifugal tube
- 333: border
- 434: cover part of transplant base
- 435: body of transplant base

## Claims

1. A system (1) for the handling of cell transplants,
which **comprises:**
(i) a continuously-operating cooler (10), which comprises at least one plate-like base (11), which comprises a cooled area (12);
(ii) a sterile positioner (30); and
(iii) a sterile cell transplant base (40);
and where the sterile positioner (30) is arranged to align the sterile cell transplant base (40) relative to the continuously-operating cooler (10) so that the cell transplant base (40) comes at the cooled area (12).

2. A system according to claim 1,
which **further comprises:**
(iv) a sterile cloth (20);
and where the sterile positioner (30) is arranged to align the sterile cell transplant base (40) at the cooled area (12) while the plate-like area (11) is covered by a sterile cloth (20) so that the cell transplant base (40) comes against the sterile cloth (20) and at the cooled area (12).

3. A system according to claim 1 or 2, **where:** alignment is implemented by means of the coding - which is advantageously a rotation coding -, shape, at least one opening and/or dimensioning of the positioner (30) and the plate-like base (11) and/or the cell transplant base (40), adapted to each other.

4. A system according to any one of the claims 1 - 3, **where** the cooler (10) comprises a cooling and adjustment system (100), which is configured to:
- keep said at least one plate-like base (11) within a temperature range that is suitable for the cell transplants in order to control the dying of cells
and/or
- keep said at least one plate-like base (11) within a temperature range that is suitable for the cell transplants in order to control the gelling speed of the extracellular matrix of the transplants.

5. A system according to any one of the preceding claims, **where:** the positioner (30) is placeable over the plate-like base (11), in which case a number of positioner (30) alignment edges (327), which are most preferably edged or contain a protrusion, have a joint effect with the alignment edges (150) of the plate-like base (11) corresponding to these in order to secure the position of the positioner (30).

6. A system according to claim 3 or 5, **where:** the positioner (30) comprises a positioning hole (326), which, when the positioner (30) is installed over the plate-like part (11), is aligned over the cooled area (12) and at which the cell transplant base (40) is placeable for cooling.

7. A system according to any one of the preceding claims, **where:** the positioner (30) and the cell transplant base (40) are separate parts of the system.

8. A system according to any one of the preceding claims 1 - 6, **where:** the positioner (30) is integrated into the cell transplant base (40).

9. A cooler (10) applicable to the cooling of cell transplants, which cooler (10) is suitable for use in a system according to any one of the preceding claims 1 - 8, **comprises:**
- at least one plate-like base (11), which comprises a cooled area (12) ;
- a cooling and adjustment system (100) for the cooling of said cooled area (12), which cooling and adjustment arrangement (100) is arranged to transfer heat from the cooled area (12) to the lower side of the plate-like base (11).

10. A cooler (10) according to claim 9, **where:** the cooling and adjustment system (100) is configured to control the cooling of the cooled area (12) and/or to adjust its temperature, advantageously by keeping said at least one cooled area (12) within a temperature range that is suitable in terms of the cell transplants in order to control the dying of cells and/or to control the gelling speed of the extracellular matrix of the transplants.

11. A cooler (10) according to claim 9 or 10, **where:** the cooling and adjustment system (100) comprises a number of thermoelectric elements or Peltier elements (113), the cold side of which is arranged to refrigerate said at least one cooled area and where the heat produced by the warm side of the elements is arranged to be transferred via a liquid cooling circuit (160) to the air or other fluid.

12. A cooler (10) according to claim 11, **where:** the cooler (10) comprises a number of blowers (121), which are arranged to remove heat from the liquid cooling circuit (160) and are placed beneath the plate-like base (11).

13. A cooler (10) according to any one of the claims 9 - 12, **where:** the cooling and adjustment system (100) comprises at least two temperature sensors (116, 117), which are arranged to monitor the temperature of the cooled area (12) or of a thermal conductor - such as a thermal conduction plate (112) - that is in a fixed thermal contact with the cooled area (12).

14. A cell transplant base (40) applicable to the cooling of cell transplants, **which is** applicable to be used in a system (1) according to any one of the preceding claims 1 - 8 and which has a cup-like recess and a bottom that is essentially flat at least in some respect, for the transfer of heat from the cell transplant base (40) via a sterile cloth (20) to the cooled area (12).

15. A cell transplant base (40) according to claim 14, which cell transplant base (40) is of metal or contains metal or some other material that conducts heat well, most advantageously so that the cell transplant base (40) has a thermal conductivity of > 14 W / (m . K), and best so that the cell transplant base (40) is of or contains steel - such as stainless steel - or aluminium.

16. A cell transplant base (40) according to claim 14 or 15, the mass of which cell transplant base (40) is < 200 g, more advantageously 30 - 150 g, best 35 - 55 g.

17. A cell transplant base (40) according to any one of the claims 14 - 16, which cell transplant base (40) comprises a cover part (434) comprising a cup-like recess, and a body (435), in which advantageously a) the body (435) and the cover part (434) have a flange-like part, which is at least partly overlapping, for receiving heat from the cover part (434) to the body (435) and/or b) have a three-dimensional shape or protrusion that facilitates the handling of the cell transplant.

18. A positioner (30) applicable to the cell transplant handling system, **which positioner (30) is** applicable to be placed on a sterile cloth (20) to be placed on the plate-like base (11) in the system according to any one of the preceding claims 1 - 8, and which is in this case also adapted to position the cell transplant base (40) at the cooled area (12) so that the cell transplant base (40) and the cooled area (12) exert a force on the sterile cloth (20) from the opposite sides of the sterile cloth (20).

19. A positioner (30) according to claim 18, which positioner (30) is so shaped that it allows both the gravitational force of the cell transplant base (40) to be essentially directed to the cooled area (12) and, correspondingly, the travel of the support force, which comes to the cell transplant base (40) via the cooled area (12) and which is opposite to the gravitational force, via the sterile cloth (30) .

20. A positioner (30) according to claim 18 or 19, a) which positioner (30) is arranged to be part of a kit, which includes sterile tools for the handling of cell transplants and/or providing epicardial autologous atrial micrograft therapy and/or b) which is part of a functional worktop and/or c) which also includes at least one cup, tube stand or cup holder.

21. A method for the cooling of cell transplants used in transplantation therapy, most advantageously in epicardial micrograft transplantation therapy, **in which method:** a system (1) according to any one of the preceding claims 1 - 8 is used so that the plate-like base (11) is covered by a sterile cloth (20), after which the sterile positioner (30) is placed over the plate-like base (11), after which the cell transplant base (40) is placed in the positioner (30), whereby the cell transplant base (40) is located at the cooled area (12) and is coolable by the cooler (10).
